# EUROPEAN PATENT SPECIFICATION

(11) **EP 3 482 210 B1**
(45) Date of publication and mention of the grant of the patent: **23.06.2021**
(21) Application number: 17755235.3
(22) Date of filing: 06.07.2017
(51) Int. Cl.: G01N 33/68

(54) **ASSAY FOR DETECTING TOTAL AND S129 PHOSPHORYLATED ALPHA-SYNUCLEIN**
VERFAHREN ZUM NACHWEIS VON GESAMTEM UND S129 PHOSPHORYLIERTEM ALPHA-SYNUKLEIN
ESSAI POUR LA DÉTECTION D'ALPHA-SYNUCLÉINE TOTALE ET PHOSPHORYLÉE SUR S129

(30) Priority: 06.07.2016 US 201662359139 P
(43) Date of publication of application: 15.05.2019
(73) Proprietor: Prothena Biosciences Limited, Dublin 2, D02 VK60 (IE)
(72) Inventor: BARBOUR, Robin, Walnut Creek, California 94598 (US); KIRIK, Deniz, 224 66 Lund (SE); LANDECK, Natalie Regina, 69427 Madau (DE)
(74) Representative: Elkington and Fife LLP
(86) International application number: PCT/IB2017/054085
(87) International publication number: WO 2018/007979

(56) References cited:
- WO-A2-2005/047860
- ANDERSON JOHN P ET AL: "Phosphorylation of Ser-129 is the dominant pathological modification of alpha-synuclein in familial and sporadic Lewy body disease", JOURNAL OF BIOLOGICAL CHEMISTRY, AMERICAN SOCIETY FOR BIOCHEMISTRY AND MOLECULAR BIOLOGY, US, vol. 281, no. 40, 6 October 2006 (2006-10-06), pages 29739-29752, XP002500217, ISSN: 0021-9258, DOI: 10.1074/JBC.M600933200 [retrieved on 2006-07-17]
- JINXIA ZHOU ET AL: "Changes in the solubility and phosphorylation of [alpha]-synuclein over the course of Parkinson's disease", ACTA NEUROPATHOLOGICA, SPRINGER, BERLIN, DE, vol. 121, no. 6, 13 March 2011 (2011-03-13), pages 695-704, XP019906863, ISSN: 1432-0533, DOI: 10.1007/S00401-011-0815-1
- MARGOT FOURNIER ET AL: "Parkin depletion delays motor decline dose-dependently without overtly affecting neuropathology in [alpha]-synuclein transgenic", BMC NEUROSCIENCE, BIOMED CENTRAL, LONDON, GB, vol. 14, no. 1, 5 November 2013 (2013-11-05), page 135, XP021165883, ISSN: 1471-2202, DOI: 10.1186/1471-2202-14-135
- NOUR K. MAJBOUR ET AL: "Oligomeric and phosphorylated alpha-synuclein as potential CSF biomarkers for Parkinson's disease", MOLECULAR NEURODEGENERATION, vol. 11, no. 1, 19 January 2016 (2016-01-19), XP055407319, DOI: 10.1186/s13024-016-0072-9
- NATALIE LANDECK ET AL: "A novel multiplex assay for simultaneous quantification of total and S129 phosphorylated human alpha-synuclein", MOLECULAR NEURODEGENERATION, vol. 11, no. 1, 22 August 2016 (2016-08-22), XP055407399, DOI: 10.1186/s13024-016-0125-0

## Description

### BACKGROUND

Alpha-synuclein brain pathology is a conspicuous feature of several neurodegenerative diseases termed synucleinopathies. Alpha-synuclein is the main component of Lewy bodies (LBs) and Lewy neurites, which are intraneuronal inclusions.

Synucleinopathies include Parkinson's disease (PD), dementia with Lewy bodies (DLB), the Lewy body variant of Alzheimer's disease (LBVAD), diffuse Lewy body disease (DLBD), multiple systems atrophy (MSA), and neurodegeneration with brain iron accumulation type-1 (NBIA-1).

Synucleinopathies are a common cause for movement disorders and cognitive deterioration in the aging population (Galasko et al., Arch. Neurol. (1994) 51:888-95). To date these disorders are neither curable nor preventable and understanding the causes and pathogenesis of PD is critical towards developing new treatments (Tanner et al., Curr. Opin. Neurol. (2000) 13:427-30). The cause for PD is controversial and multiple factors have been proposed to play a role, including various neurotoxins and genetic susceptibility factors.

Several studies have shown that alpha-synuclein plays a central role in PD pathogenesis because: (1) this protein accumulates in LBs (Spillantini et al., Nature (1997) 388:839-40; Takeda et al., J. Pathol. (1998) 152:367-72; Wakabayashi et al., Neurosci. Lett. (1997) 239:45-8), (2) mutations in the alpha-synuclein gene co-segregate with rare familial forms of Parkinsonism (Kruger et al., Nature Gen. (1998) 18:106-8; Polymeropoulos et al., Science (1997) 276:2045-7) and, (3) its overexpression in transgenic mice (Masliah et al., Science (2000) 287:1265-9) and Drosophila (Feany et al., Nature (2000) 404:394-8) mimics several pathological aspects of PD. Thus, the fact that accumulation of alpha-synuclein in the brain is associated with similar morphological and neurological alterations in species as diverse as humans, mice, and flies suggests that this molecule contributes to the development of PD.

LBs were consistently found to contain a large amount of accumulated alpha-synuclein (Spillantini et al. National Acad Sciences 95: 6469-6473 (1998)). Analysis of abnormally processed and aggregated asyn from patients have revealed different posttranslational modifications, including phosphorylation, nitration, ubiquitination and C-terminal truncation of the protein (Giasson et al. Science 290: 985-989 (2000); Baba et al. Am J Pathol. 152: 879-884 (1998); Fujiwara et al. Nat Cell Biol. 4: 160-164 (2002)). Most of the alpha-synuclein accumulated in brains of patients is indeed phosphorylated especially at serine 129 (pS129 h-asyn) (Fujiwara et al. Nat Cell Biol. 4: 160-164 (2002); Anderson et al. J Biol Chem. 281: 29739-29752 (2006)). These findings lead to the development of independent assays monitoring total alpha-synuclein levels as well as its oligomeric and phosphorylated states, not only in brain tissue but also in cerebrospinal fluid (CSF) and blood samples (El-Agnaf et al. FASEB J. 20: 419-425(2006); Hong et al. Brain 133: 713-726(2010); Emmanouilidou et al. PLoS ONE. (2001); Bidinosti et al. J Biol Chem. 287(40):33691-705(2012); Kruse et al. Neurobiology of Aging. 36: 2587-2596 (2015); Mollenhauer et al. Experimental Neurology. 213: 315-325(2008)). WO-A-2005/047860 describes methods for detecting alpha-synuclein, identifies preferred epitopes of alpha-synuclein for use in such detection, and provides antibodies specifically binding to such epitopes. Although assays for alpha-synuclein as a wet biomarker have been developed, particularly to quantify total alpha-synuclein levels for correlation with disease onset and progression, only four assays have been developed for quantifying S129 phosphorylated alpha-synuclein (Anderson et al. J Biol Chem. 281: 29739-29752 (2006); Swirski et al. Alzheimers Res Ther.6: 77(2014); Foulds et al. FASEB J. 25: 4127-4137 (2011); Wang et al. Science Translational Medicine. 4: 121(2012)). Notably, none of the above methods implemented a duplex assay platform to measure total alpha-synuclein and S129 phosphorylated alpha-synuclein in a single well.

The invention provides a novel assay which allows simultaneous, sensitive and specific analysis of total alpha-synuclein human asyn (h-asyn) on the same molecule hence improving accuracy as well as saving time, consumables and samples.

### SUMMARY OF THE CLAIMED INVENTION

The invention provides an in vitro method of simultaneously detecting total alpha-synuclein and S129 phosphorylated (pS129) alpha-synuclein, comprising: contacting a sample with: (i) a donor complex comprising an antibody that specifically binds to alpha-synuclein linked to a support and a donor label, which is light excitable to activate oxygen to produce singlet oxygen; (ii) a first acceptor complex for detecting pS129 alpha-synuclein comprising an antibody that binds to pS129 alpha-synuclein with an association constant at least five times higher for pS129 alpha-synuclein than for unphosphorylated alpha-synuclein, linked to a support and a first acceptor label; wherein if pS129 alpha-synuclein is present in the sample, the antibodies of the donor complex and first acceptor complex bind to the pS129 alpha-synuclein forming a first sandwich in which the donor label is light excitable to produce singlet oxygen to activate the first acceptor label to produce a first signal; (iii) a second acceptor complex for detecting total alpha-synuclein comprising an antibody that binds to alpha-synuclein regardless of S129 phosphorylation, the antibody being linked to a support and a second acceptor label; wherein if total alpha-synuclein is present in the sample, the antibodies of the donor complex and second acceptor complex bind to total alpha-synuclein forming a second sandwich in which the donor label is light excitable to produce singlet oxygen to activate the second acceptor label to produce a second signal; (b) exciting the donor label with a light to activate oxygen to produce singlet oxygen, thereby producing the first signal if pS129 alpha-synuclein is present and the second signal if total alpha-synuclein is present; and (c) detecting the first and second signals, if any, to indicate presence of pS129 alpha-synuclein and total alpha synuclein, respectively. Optionally, the second signal is detected before the first signal. Optionally, the contacting of the sample with the donor complex comprises contacting the sample with the support linked to the donor label of the donor complex, wherein the support is further linked to streptavidin and the antibody of the donor complex, wherein the antibody is linked to biotin, wherein the streptavidin binds to the biotin forming the donor complex in the sample to thereby contact the sample. Optionally the sample is contacted with the first and second acceptor complexes and the antibody of the donor complex linked to biotin, incubating the sample, and thereafter contacting the sample with the support further linked to streptavidin.

Optionally, the contact of the sample with the donor complex comprises contacting the sample with the support linked to the donor label and a biotin label and the antibody linked to streptavidin, wherein the streptavidin binds to the biotin forming the donor complex which thereby contacts the sample.

Optionally, the method is performed qualitatively. Optionally, the method is performed quantitatively in which the first signal indicates an amount of pS129 alpha-synuclein and the second signal indicates an amount of total alpha-synuclein. Optionally, the donor complex, first acceptor complex and second acceptor complex are contacted with the sample simultaneously. Optionally, the first and second acceptor complexes are contacted with the sample before the donor complex.

Optionally, the antibody of the first acceptor complex specifically binds to an epitope of phosphorylated synuclein within residues 123-134. Optionally, the antibody of the first acceptor complex is 11A5 (ATCC Accession No. PTA-8222).

Optionally, the antibody of the donor complex specifically binds to an epitope within residues 103-108 of alpha-synuclein. Optionally, the antibody of the donor complex is 4B12 (available for purchase from BioLegend®, 9727 Pacific Heights Blvd., San Diego, CA 92121, www.biolegend.com, Catalog #'s 807801, 807802 or 807803, previously Covance Catalog # SIG-39730). Optionally, the antibody of the donor complex specifically binds to an epitope within residues 40-55 of alpha-synuclein. Optionally, the antibody of the donor complex is 23E8 (ATCC Accession No. PTA-122711). Optionally, the antibody of the donor complex specifically binds to an epitope within residues 91-97 of alpha-synuclein. Optionally, the antibody of the donor complex is 1H7 (ATCC Accession No. PTA-8220). Optionally, the antibody of the donor complex specifically binds to an epitope within residues 109-120 of alpha-synuclein. Optionally, the antibody of the donor complex is 5C12 (ATCC Accession No. PTA-9197). Optionally, the antibody of the donor complex specifically binds to an epitope within residues 115-122 of alpha-synuclein. Optionally, the antibody of the donor complex is LB509 (available for purchase from BioLegend®, 9727 Pacific Heights Blvd., San Diego, CA 92121, www.biolegend.com, Catalog #'s 807701, 807702, 807703 or 80774, previously Covance Catalog # SIG-39725 or SIG-39726). Optionally, the antibody of the donor complex is 4B12, LB509, 1H7, 5C12 or 23E8.

The antibody of the second acceptor complex can be any of a number of antibodies that bind to alpha-synuclein. In some methods of the invention, the epitope of the donor complex antibody is different from the epitope of the second complex acceptor antibody. Some of such epitopes are distinct (i.e., do not overlap with or block each other). For example, the antibody of the donor complex specifically binds to an epitope within 40-55, 91-99, 103-108, 109-122 or 115-122 of alpha-synuclein and the antibody of the second acceptor complex specifically binds to an epitope within 40-55, 91-99, 103-108, 109-122 or 115-122, provided that the epitope specifically bound by the antibody of the donor complex is distinct from the epitope specifically bound by the antibody of the second acceptor complex. For example, the donor complex antibody can be 23E8, 1H7, 4B12, 5C12 or LB509 and the second acceptor complex can be 23E8, 1H7, 4B12, 5C12 or LB509, provided that the donor complex antibody and the second acceptor complex antibody are different antibodies. In some methods, the antibody of the second acceptor complex specifically binds to an epitope within residues 115-122 of alpha-synuclein. Optionally, the antibody of the second acceptor complex is LB509. Optionally, the antibody of the second acceptor complex specifically binds to an epitope within residues 91-97 of alpha-synuclein. Optionally, the antibody of the second acceptor complex is 1H7 Optionally, the antibody of the second acceptor complex specifically binds to an epitope within residues 109-122 of alpha-synuclein. Optionally, the antibody of the second acceptor complex is 5C12. Optionally, the antibody of the second acceptor complex specifically binds to an epitope within residues 40-55 of alpha-synuclein. Optionally, the antibody of the second acceptor complex is 23E8.

Optionally, the antibody of the second acceptor complex is 4B12, LB509, 1H7, 5C12 or 23E8. Optionally, the antibody of the donor complex is 4B12, and the antibody of the second acceptor complex is LB509, 1H7, 5C12, or 23E8. Optionally, the antibody of the donor complex is 4B12 and the antibody of the second acceptor complex is LB509. Optionally, the antibody of the donor complex is 23E8 and the antibody of the second acceptor complex is LB509, 1H7, 5C12, or 23E8. Optionally, the antibody of the donor complex is 23E8 and the antibody of the second acceptor complex is 1H7. Optionally, the antibody of the donor complex is 5C12 and the antibody of the second acceptor complex is 23E8 or 1H7.

Optionally, the supports of the donor complex, the first acceptor complex, and the second acceptor complex are beads. Optionally, the first acceptor label comprises Europium, Terbium, Samarium or Gadolinium. Optionally, the second acceptor label comprises Europium, Terbium, Samarium or Gadolinium. Optionally, the first acceptor label comprises Europium and the second acceptor label comprises Terbium.

Optionally, the sample is a tissue from a human. Optionally, the sample is from a transgenic mouse with a transgene expressing human alpha-synuclein. Optionally, the sample is a body fluid. Optionally, the sample is cerebrospinal fluid (CSF) of a human. Optionally, the sample is a brain, skin, or other organ homogenate of a human, a wild type or transgenic animal. Optionally, the sample is a medium used to culture cells. Optionally, the cells express recombinant human alpha-synuclein.

### BRIEF DESCRIPTION OF THE DRAWINGS

Figs. 1A-D: show the characterization of total human alpha-synuclein assay.
Figs. 2A-D: show the characterization of S129 phosphorylated human alpha-synuclein specific assay.
Fig. 3: show the amount of phosphorylated h-asyn using isoelectric focusing gels.
Figs. 4A-F: show the duplex assay performance.
Fig. 5: show the comparison of signal intensity of uniplex and duplex conditions.
Figs. 6A-F: show the characterization of total and phosphorylated S129 human alpha-synuclein duplex assay.
Fig. 7: show S129 specific phosphorylation of human alpha-synuclein using PLK2 in vitro.

### DEFINITIONS

The phrase that an antibody "specifically binds" to a target refers to a binding reaction which is determinative of the presence of the antibody in the presence of a heterogeneous population of other biologics. Thus, under designated immunoassay conditions, a specified molecule binds preferentially to a particular target and does not bind in a significant amount to other biologics present in the sample. Specific binding of an antibody to a target under such conditions requires the antibody be selected for its specificity to the target. Specific binding between two entities means an affinity of at least 10⁶, 10⁷, 10⁸, 10⁹ or 10¹⁰ M⁻¹. Affinities greater than 10⁸ M⁻¹ are preferred. Lack of specific binding means binding to a target indistinguishable from an irrelevant control antibody and/or an affinity of less than 10⁶ M⁻¹.

The term "antibody" includes intact antibodies and binding fragments thereof. Typically, fragments compete with the intact antibody from which they were derived for specific binding to an antigen fragment including separate heavy chains, light chains Fab, Fab' F(ab')2, Fabc, and Fv. Fragments are produced by recombinant DNA techniques, or by enzymatic or chemical separation of intact immunoglobulins. The term "antibody" also includes one or more immunoglobulin chains that are chemically conjugated to, or expressed as, fusion proteins with other proteins. The term "antibody" also includes bispecific antibody. A bispecific or bifunctional antibody is an artificial hybrid antibody having two different heavy/light chain pairs and two different binding sites. Bispecific antibodies can be produced by a variety of methods including fusion of hybridomas or linking of Fab' fragments. *See, e.g.,* Songsivilai & Lachmann, Clin. Exp. Immunol. 79:315-321 (1990); Kostelny et al., J. Immunol. 148, 1547-1553 (1992).

Antibodies of the invention are typically substantially pure from undesired contaminant. This means that an agent is typically at least about 50% w/w (weight/weight) purity, as well as being substantially free from interfering proteins and contaminants. Sometimes the antibodies are at least about 80% w/w and, more preferably at least 90 or about 95% w/w purity. However, using conventional protein purification techniques, homogeneous antibodies of at least 99% w/w can be obtained.

The term "epitope" or "antigenic determinant" refers to a site on an antigen to which B and/or T cells respond. B-cell epitopes can be formed both from contiguous amino acids or noncontiguous amino acids juxtaposed by tertiary folding of a protein. Epitopes formed from contiguous amino acids or post-translationally modified amino acids are typically retained on exposure to denaturing solvents whereas epitopes formed by tertiary folding are typically lost on treatment with denaturing solvents. An epitope typically includes at least 3, but generally speaking 5-10 amino acids in a unique spatial conformation. Methods of determining spatial conformation of epitopes include, for example, x-ray crystallography and 2-dimensional nuclear magnetic resonance. *See, e.g.,* Epitope Mapping Protocols in Methods in Molecular Biology, Vol. 66, Glenn E. Morris, Ed. (1996). Antibodies that recognize the same epitope can be identified in a simple immunoassay showing the ability of one antibody to block the binding of another antibody to a target antigen.

The term "body fluid" refers to those fluids of a mammalian host which is suspected contain measurable amounts of alpha-synuclein or fragments thereof, specifically including blood, cerebrospinal fluid (CSF), brain or any other organ interstitial fluid (ISF), urine, saliva, aqueous humour, and peritoneal fluid. The term "blood" refers to whole blood, as well as blood plasma and serum.

A synucleinopathic disease means a disease characterized by Lewy bodies, Lewy neurites or other deposits of alpha-synuclein.

Qualitative assay detects presence or absence of an analyte. A quantitative assay detects not only presence or absence of the analyte but if present provides an absolute or relative amount of the analyte.

Compositions or methods "comprising" one or more recited elements may include other elements not specifically recited. For example, a composition that comprises alpha-synuclein peptide encompasses both an isolated alpha-synuclein peptide and alpha-synuclein peptide as a component of a larger polypeptide sequence.

The term "total alpha-synuclein" refers to alpha-synuclein regardless of whether it is phosphorylated at S129. If alpha-synuclein is from a human, it is referred to as "h-asyn." If alpha-synuclein is from a mouse, it is referred to as "m-asyn." Thus, determination of presence or amount of total alpha-synuclein in a sample can reflect the presence or amount of alpha-synuclein phosphorylated at S129, alpha-synuclein not phosphorylated at S129 or both.

A sample refers to a test aliquot that may contain pS129 or total alpha-synuclein or both. Such samples can be obtained from patients, cell cultures or transgenic animals, among other sources as discussed further below. A sample can be subject to further processing before analysis, such as the removal of irrelevant components, or addition of solvents, buffers and so forth.

### DETAILED DESCRIPTION

The invention provides methods for detecting total alpha-synuclein and pS129 alpha-synuclein in a sample by a single assay, thereby allowing for accurate calculation of the proportion of alpha-synuclein molecules phosphorylated at S129. The assay requires no washing and can be completed with a short incubation time. Thus the assay can be incorporated into high throughput screens, allowing for cost efficient runs and using lower amounts of sample material than running separate uniplex assays.

### I. Antibodies used in Detection

The present methods detect total alpha-synuclein and S129 phosphorylated (pS129) alpha-synuclein using a donor complex, a first acceptor complex, and a second acceptor complex. The donor complex comprises an antibody that specifically binds to alpha-synuclein linked to a support and a donor label. The antibody binds to an epitope different from and preferably non-overlapping with the epitopes bound by the antibodies of first and second acceptor complexes to be discussed below. The antibody binds regardless of whether alpha-synuclein is phosphorylated at S129. An antibody binding regardless of phosphorylation at S129 means an antibody with association constants for alpha-synuclein with or without phosphorylation at S129 differing by no more than a factor of 1.5 and preferably indistinguishable (other than insignificant experimental error). The donor label is light excitable to activate oxygen to produce singlet oxygen. Optionally, the antibody of the donor complex specifically binds to an epitope within residues 103-108 of alpha-synuclein. The 4B12 antibody is an example of such an antibody in the donor complex. Optionally, the antibody of the donor complex specifically binds to an epitope within residues 40-55 of alpha-synuclein. The 23E8 antibody is an example of such an antibody in the donor complex. The 23E8 antibody has been deposited as ATCC Accession No. PTA-122711. Optionally, the antibody of the donor complex specifically binds to an epitope within residues 91-97 of alpha-synuclein. The 1H7 antibody is an example of such an antibody in the donor complex. The 1H7 antibody has been deposited as ATCC Accession No. PTA-8220. Optionally, the antibody of the donor complex specifically binds to an epitope within residues 109-120 of alpha-synuclein. The 5C12 antibody is an example of such an antibody in the donor complex. The 5C12 antibody has been deposited as ATCC accession no. PTA-9197. Optionally, the antibody of the donor complex specifically binds to an epitope within residues 40-55 of alpha-synuclein. The 23E8 antibody is an example of such an antibody in the donor complex. The 23E8 antibody has been deposited as ATCC Accession No. PTA-122711.

The first acceptor complex for detecting pS129 alpha-synuclein comprises an antibody that preferentially binds to pS129 alpha-synuclein linked to a support and a first acceptor label. Preferential binding means an association constant at least five times higher for pS129 alpha-synuclein than unphosphorylated alpha-synuclein. Optionally, the association constant is at least ten times higher for pS129 alpha-synuclein than unphosphorylated alpha-synuclein. Optionally, the antibody lacks specific binding to unphosphorylated alpha-synuclein. The 11A5 antibody is an example of a suitable antibody in the first acceptor complex. The 11A5 antibody has been deposited as ATCC Accession No. PTA-8222.

The second acceptor complex for detecting total alpha-synuclein comprises an antibody that binds to alpha-synuclein regardless of S129 phosphorylation. The antibody of the second acceptor complex is linked to a support and a second acceptor label. The antibody of the second acceptor complex can be any of a number of antibodies that bind to alpha-synuclein. The antibody of the second acceptor complex binds to an epitope different than the epitopes bound by the antibodies of the donor complex and first acceptor complex, and binds to alpha-synuclein regardless of whether S129 is phosphorylated. Some of such epitopes are distinct (i.e., are non-overlapping with and/or or block each other). For example, the antibody of the donor complex specifically binds to an epitope within 40-55, 91-99, 103-108, 109-122 or 115-122 of alpha-synuclein and the antibody of the second acceptor complex specifically binds to an epitope within 40-55, 91-99, 103-108, 109-122 or 115-122, provided that the epitope specifically bound by the antibody of the donor complex is distinct from the epitope specifically bound by the antibody of the second acceptor complex. For example, the donor complex antibody can be 23E8, 1H7, 4B12, 5C12 or LB509 and the second acceptor complex can be 23E8, 1H7, 4B12, 5C12 or LB509, provided that the donor complex antibody and the second acceptor complex antibody are different antibodies. In some methods, the antibody of the second acceptor complex specifically binds to an epitope within residues 115-122 of alpha-synuclein. The LB509 antibody is an example of such an antibody in the second acceptor complex. Optionally, the antibody of the second acceptor complex specifically binds to an epitope within residues 91-97 of alpha-synuclein. The 1H7 antibody is an example of such an antibody in the second acceptor complex. The 1H7 antibody has been deposited as ATCC Accession No. PTA-8220. Optionally, the antibody of the second acceptor complex specifically binds to an epitope within residues 109-122 of alpha-synuclein. The 5C12 antibody is an example of such an antibody in the second acceptor complex. Optionally, the antibody of the second acceptor complex specifically binds to an epitope within residues 40-55 of alpha-synuclein. The 23E8 antibody is an example of such an antibody in the second acceptor complex.

When an antibody is said to bind to an epitope within specified residues, such as alpha-synuclein 40-55, for example, what is meant is that the antibody specifically binds to a polypeptide consisting of the specified residues (i.e., alpha-synuclein 40-55 in this an example). Such an antibody does not necessarily contact every residue within alpha-synuclein 40-55. Nor does every single amino acid substitution or deletion within alpha-synuclein 40-55 necessarily significantly affect binding affinity. Epitope specificity of an antibody can be determined, for example, by testing a collection of overlapping peptides of about 15 amino acids spanning the sequence of alpha-synuclein and differing in increments of a small number of amino acids (e.g., 3 amino acids). The peptides are immobilized within the wells of a microtiter dish. Immobilization can be effected by biotinylating one terminus of the peptides. Optionally, different samples of the same peptide can be biotinylated at the N and C terminus and immobilized in separate wells for purposes of comparison. Such is particularly useful for identifying end-specific antibodies. An antibody is screened for specific binding to each of the various peptides. The epitope is defined as occurring within a segment of amino acids that is common to all peptides to which the antibody shows specific binding. Overlapping epitopes mean epitopes that share some but not all residues. For example, an epitope of residues 140-155 overlaps with an epitope of residues 154-160.

### i. General Characteristics of Immunoglobulins

The basic antibody structural unit is known to comprise a tetramer of subunits. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function.

Light chains are classified as either kappa or lambda. Heavy chains are classified as gamma, mu, alpha, delta, or epsilon, and define the antibody's isotype as IgG, IgM, IgA, IgD and IgE, respectively. Within light and heavy chains, the variable and constant regions are joined by a "J" region of about 12 or more amino acids, with the heavy chain also including a "D" region of about 10 more amino acids. (*See* generally, Fundamental Immunology, Paul, W., ed., 2nd ed. Raven Press, N.Y., 1989, Ch. 7 .

The variable regions of each light/heavy chain pair form the antibody binding site. Thus, an intact antibody has two binding sites. Except in bifunctional or bispecific antibodies, the two binding sites are the same. The chains all exhibit the same general structure of relatively conserved framework regions (FR) joined by three hypervariable regions, also called complementarity determining regions or CDRs. The CDRs from the two chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each domain is in accordance with the definitions of Kabat, Sequences of Proteins of Immunological Interest (National Institutes of Health, Bethesda, Md., 1987 and 1991); Chothia & Lesk, J. Mol. Biol. 196:901-917 (1987); or Chothia et al., Nature 342:878-883 (1989).

### ii. Production of Nonhuman Antibodies

Mouse or other non-human antibodies can be produced by conventional hybridoma technology. The desired binding specificity can be imparted by selection of the immunogen and/or the screening approach. For generating antibodies with an epitope specificity between residues 40 and 55, a fragment of alpha-synuclein consisting of these residues (i.e., 40-55) can be used an immunogen or a longer fragment including these residues up to full-length alpha-synuclein. Antibodies can be screened by binding to overlapping peptides as described above. For producing an antibody preferentially binding to S129 phosphorylated alpha-synuclein (pS129 alpha-synuclein), full length pS129 alpha-synuclein or a fragment thereof including residue 129 and sufficient residues either side to constitute an epitope (e.g., 3-15 contiguous residues including residue 129) can be used as the immunogen. Antibodies are screened for preferential binding to pS129 alpha-synuclein against unphosphorylated alpha-synuclein.

Chimeric and humanized antibodies have the same or similar binding specificity and affinity as a mouse or other nonhuman antibody that provides the starting material for construction of a chimeric or humanized antibody. Chimeric antibodies are antibodies whose light and heavy chain genes have been constructed, typically by genetic engineering, from immunoglobulin gene segments belonging to different species. For example, DNA encoding the variable domains of a mouse antibody can be sequenced, and DNA construct(s) encoding the variable domains joined to human constant (C) segments, such as IgG1 and IgG4 constructed. The constructs are then expressed to produce the antibody Human isotype IgG1 is preferred. In some methods, the isotype of the antibody is human IgG1. IgM antibodies can also be used in some methods. A typical chimeric antibody is thus a hybrid protein consisting of the V or antigen-binding domain from a mouse antibody and the C or effector domain from a human antibody.

Humanized antibodies have variable region framework residues substantially from a human antibody or consensus of human antibodies (termed an acceptor antibody) and some and usually all six complementarity determining regions substantially or entirely from a mouse-antibody, (referred to as the donor immunoglobulin). *See,* Queen et al., Proc. Natl. Acad. Sci. USA 86:10029-10033 (1989), WO 90/07861, U.S. Pat. No. 5,693,762, U.S. Pat. No. 5,693,761, U.S. Pat. No. 5,585,089, U.S. Pat. No. 5,530,101, and Winter, U.S. Pat. No. 5,225,539. The constant region(s), if present, are also substantially or entirely from a human immunoglobulin. The human variable domains are usually chosen from human antibodies whose framework sequences exhibit a high degree of sequence identity with the murine variable region domains from which the CDRs were derived. The heavy and light chain variable region framework residues can be derived from the same or different human antibody sequences. The human antibody sequences can be the sequences of naturally occurring human antibodies or can be consensus sequences of several human antibodies. *See* Carter et al., WO 92/22653. Certain amino acids from the human variable region framework residues are selected for substitution based on their possible influence on CDR conformation and/or binding to antigen. Investigation of such possible influences is by modeling, examination of the characteristics of the amino acids at particular locations, or empirical observation of the effects of substitution or mutagenesis of particular amino acids.

For example, when an amino acid differs between a murine variable region framework residue and a selected human variable region framework residue, the human framework amino acid should usually be substituted by the equivalent framework amino acid from the mouse antibody when it is reasonably expected that the amino acid: (1) noncovalently binds antigen directly, (2) is adjacent to a CDR region, (3) otherwise interacts with a CDR region (e.g. is within about 6 Å of a CDR region), or (4) participates in the VL-VH interface.

Other candidates for substitution are acceptor human framework amino acids that are unusual for a human immunoglobulin at that position. These amino acids can be substituted with amino acids from the equivalent position of the mouse donor antibody or from the equivalent positions of more typical human immunoglobulins. The variable region frameworks of humanized immunoglobulins usually show at least 85% sequence identity to a human variable region framework sequence or consensus of such sequences.

### iii. Human Antibodies

Human antibodies against alpha-synuclein are provided by a variety of techniques described below. Human antibodies can also be screened for a particular epitope specificity by using only a fragment of alpha-synuclein as the immunogen, and/or by screening antibodies against a collection of deletion mutants of alpha-synuclein. Human antibodies preferably have isotype specificity human IgG1. Several methods are available for producing human antibodies including the trioma method, Oestberg et al., Hybridoma 2:361-367 (1983); Oestberg, U.S. Pat. No. 4,634,664; and Engleman et al., U.S. Pat. No. 4,634,666; transgenic non-human mammals described in detail by, e.g., Lonberg et al., WO93/1222, U.S. Pat. No. 5,877,397, U.S. Pat. No. 5,874,299, U.S. Pat. No. 5,814,318, U.S. Pat. No. 5,789,650, U.S. Pat. No. 5,770,429, U.S. Pat. No. 5,661,016, U.S. Pat. No. 5,633,425, U.S. Pat. No. 5,625,126, U.S. Pat. No. 5,569,825, U.S. Pat. No. 5,545,806, Nature 148, 1547-1553 (1994), Nature Biotechnology 14, 826 (1996), Kucherlapati, WO 91/10741; and phage display methods See, e.g., Dower et al., WO 91/17271 and McCafferty et al., WO 92/01047, U.S. Pat. No. 5,877,218, U.S. Pat. No. 5,871,907, U.S. Pat. No. 5,858,657, U.S. Pat. No. 5,837,242, U.S. Pat. No. 5,733,743 and U.S. Pat. No. 5,565,332.

### iv. Selection of Constant Region

The heavy and light chain variable regions of chimeric, humanized, or human antibodies can be linked to at least a portion of a human constant region. The choice of constant region depends, in part, whether antibody-dependent complement and/or cellular mediated toxicity is desired. For example, isotopes IgG1 and IgG3 have complement activity and isotypes IgG2 and IgG4 do not. Choice of isotype can also affect passage of antibody into the brain. Human isotype IgG1 is preferred. Light chain constant regions can be lambda or kappa. Antibodies can be expressed as tetramers containing two light and two heavy chains, as separate heavy chains, light chains, as Fab, Fab' F(ab')2, and Fv, or as single chain antibodies in which heavy and light chain variable domains are linked through a spacer.

### v. Expression of Recombinant Antibodies

Chimeric, humanized and human antibodies are typically produced by recombinant expression. Recombinant polynucleotide constructs typically include an expression control sequence operably linked to the coding sequences of antibody chains, including naturally associated or heterologous promoter regions. Preferably, the expression control sequences are eukaryotic promoter systems in vectors capable of transforming or transfecting eukaryotic host cells. Once the vector has been incorporated into the appropriate host, the host is maintained under conditions suitable for high level expression of the nucleotide sequences, and the collection and purification of the crossreacting antibodies.

These expression vectors are typically replicable in the host organisms either as episomes or as an integral part of the host chromosomal DNA. Commonly, expression vectors contain selection markers, *e.g*., ampicillin-resistance or hygromycin-resistance, to permit detection of those cells transformed with the desired DNA sequences.

*E. coli* is one prokaryotic host particularly useful for cloning the DNA sequences of the present invention. Microbes, such as yeast are also useful for expression. *Saccharomyces* is a preferred yeast host, with suitable vectors having expression control sequences, an origin of replication, termination sequences and the like as desired. Typical promoters include 3-phosphoglycerate kinase and other glycolytic enzymes. Inducible yeast promoters include, among others, promoters from alcohol dehydrogenase, isocytochrome C, and enzymes responsible for maltose and galactose utilization.

Mammalian cells are a preferred host for expressing nucleotide segments encoding immunoglobulins or fragments thereof. *See* Winnacker, From Genes to Clones, (VCH Publishers, NY, 1987). A number of suitable host cell lines capable of secreting intact heterologous proteins have been developed in the art, and include CHO cell lines, various COS cell lines, HeLa cells, L cells, human embryonic kidney cell, and myeloma cell lines. Preferably, the cells are nonhuman. Expression vectors for these cells can include expression control sequences, such as an origin of replication, a promoter, an enhancer (Queen et al., Immunol. Rev. 89:49 (1986)), and necessary processing information sites, such as ribosome binding sites, RNA splice sites, polyadenylation sites, and transcriptional terminator sequences. Preferred expression control sequences are promoters derived from endogenous genes, cytomegalovirus, SV40, adenovirus, bovine papillomavirus, and the like. *See* Co et al., J. Immunol. 148:1149 (1992).

Alternatively, antibody coding sequences can be incorporated in transgenes for introduction into the genome of a transgenic animal and subsequent expression in the milk of the transgenic animal (*see, e.g.,* U.S. Pat. No. 5,741,957, U.S. Pat. No. 5,304,489, U.S. Pat. No. 5,849,992). Suitable transgenes include coding sequences for light and/or heavy chains in operable linkage with a promoter and enhancer from a mammary gland specific gene, such as casein or beta lactoglobulin.

The vectors containing the DNA segments of interest can be transferred into the host cell by well-known methods, depending on the type of cellular host. For example, calcium chloride transfection is commonly utilized for prokaryotic cells, whereas calcium phosphate treatment, electroporation, lipofection, biolistics or viral-based transfection can be used for other cellular hosts. Other methods used to transform mammalian cells include the use of polybrene, protoplast fusion, liposomes, electroporation, and microinjection (*see generally,* Sambrook et al., supra). For production of transgenic animals, transgenes can be microinjected into fertilized oocytes, or can be incorporated into the genome of embryonic stem cells, and the nuclei of such cells transferred into enucleated oocytes.

Once expressed, antibodies can be purified according to standard procedures of the art, including HPLC purification, column chromatography, and gel electrophoresis and the like (*see generally,* Scopes, Protein Purification (Springer-Verlag, NY, 1982).

### II. Alpha-Synuclein

Alpha-synuclein was originally identified in human brains as the precursor protein of the non-.beta.-amyloid component of (NAC) of AD plaques. (Ueda et al., Proc. Natl. Acad. Sci. U.S.A. 90 (23): 11282-11286 (1993)). Alpha-synuclein, also termed the precursor of the nοn-Aβ component of AD amyloid (NACP), is a peptide of 140 amino acids. Full-length alpha-synuclein has the amino acid sequence:
(SEQ ID NO: 1) MDVFMKGLSKAKEGVVAAAEKTKQGVAEAAGKTKEGVLYVGSKTKEGVVH GVATVAEKTKEQVTNVGGAVVTGVTAVAQKTVEGAGSIAAATGFVKKDQL GKNEEGAPQEGILEDMPVDPDNEAYEMPSEEGYQDYEPEA (Ueda et al., Ibid.; GenBank accession number: P37840).

Unless otherwise indicated, reference to alpha-synuclein means the natural human amino acid sequence indicated above as well as natural allelic and species variants thereof, including full-length forms and fragments thereof found in samples being analyzed, as well as forms having undergone posttranslational modification, such as phosphorylation. Fragments or variants of alpha-synuclein are numbered as in the exemplified sequences such that aligned residues are allocated the same number.

### III. Assays for Alpha Synuclein

Alpha-synuclein can be detected by an immunoassay in which an antibody of a donor complex is linked to a support and a donor label, an antibody of a first acceptor complex is linked to a support and a first acceptor label, and an antibody of a second acceptor complex is linked to a support and a second acceptor label. The antibody of the first acceptor complex specifically binds to pS129 alpha-synuclein. So if pS129 alpha-synuclein is present in the sample, the antibodies of the donor complex and the first acceptor complex can bind pS129 alpha-synuclein at the same time forming a first sandwich, in which the respective antibodies are held in sufficient proximity that the donor label is light excitable to produce singlet oxygen to activate the first acceptor label to produce a first signal. The antibodies of the donor complex and the second acceptor complex have different specificities from each other (and the antibody of the first acceptor complex), so both can bind to alpha-synuclein at the same time forming a second sandwich, in which the donor and second acceptor labels are held in sufficient proximity that the donor label is light excitable to produce singlet oxygen to activate the second acceptor label to produce a second signal.

Samples can be contacted with complexes in preformed state or can be contacted with components of a complex which assemble in the sample. The latter can be achieved by labelling an antibody and a support with a binding pair, such as biotin streptavidin, such that the binding of biotin to streptavidin attaches the antibody to a support in the sample. In some methods, the acceptor complexes are supplied preformed and the donor complex is supplied with the antibody labelled with biotin and the support labelled with streptavidin such that the donor complex forms in the sample thereby contacting the sample.

The complexes can be contacted with the sample simultaneously or sequentially. A preferred order of contacting is that the first and second acceptor complexes are contacted with the sample together with the antibody of the donor complex. After incubation to allow the respective antibodies to bind to their targets (if present), the support and linked donor label of the donor complex are supplied and a further incubation is performed to allow the antibody of the donor complex to associate with its support via pairing of binding moieties, such as streptavidin and biotin.

Samples are incubated to allow antibodies to bind to their targets if present. The incubation time can range from *e.g.,* 10 min to 5 hr, or 30-120 min. A preferred incubation time is 1hr. Such incubation is preferably performed in the dark.

If donor complexes are initially supplied as components with the antibody being supplied first followed by the support, a further incubation is performed after supplying the support for the antibody to bind to the support via association of a binding pair such as streptavidin-biotin. The time for this additional incubation can range from, e.g., 10 min to 2 hrs. The incubation is also preferably conducted in the dark.

After the incubation, light is directed at the sample to excite the donor label to activate oxygen to produce singlet oxygen. If pS129 alpha-synuclein is present a first signal is generated, and if total alpha-synuclein is present a second signal is present. The signals can be detected simultaneously or sequentially. Preferably, the second signal is detected before the first signal. The detected first signal indicates presence and/or an amount of pS129 alpha-synuclein in the sample. Likewise, he detected second signal indicates presence and/or an amount of total alpha-synuclein in the sample. Such amounts can be relative or absolute. For a given group of a donor complex, a first acceptor complex, and a second acceptor complex, standard curves of total alpha-synuclein and pS129 alpha-synuclein can be acquired by serially diluting samples containing known concentrations of unphosphorylated alpha-synuclein and S129 phosphorylated alpha-synuclein. The standard curves are then fitted on signal measured and corresponding log transformed alpha-synuclein concentration values by a software program.

Suitable donor labels for use in the above methods are light excitable molecules which activates oxygen to produce singlet oxygen. Exemplary donor labels include, without limitation, acetone, benzophenone, chlorophylls, 9,10-dibromoanthracene, eosin, fullerenes, methylene blue, metalloporphyrins, such as hematoporphyrin, phthalocyanines, such as Al3⁺phthalocyanines, Cu²⁺ phthalocyanines and Zn²⁺ phthalocyanines, rose Bengal and 9-thioxanthone. These and other donor labels are described in U.S. Pat. Nos. 5,340,716; 5,516,636; 5,536,834; 5,709,994; 5,763,602; 6,251,581; 6,406,667; 6,797,481; 7,033,775; 7,101,682; N. J. Turro, "Molecular Photochemistry", page 132, W. A. Benjamin Inc., New York 1965; Ullman, et al., Proc. Natl. Acad. Sci. USA 91, 5426-5430 (1994); Strong et al, Ann. New York Acad. Sci., 745: 297-320 (1994); Martin et al, Methods Enzymol., 186: 635-645 (1990);Yarmush et al, Grit. Rev. Therapeutic Drug Carrier Syst., 10: 197-252 (1993); Wohrle, Chimia, 45: 307-310 (1991); Thetford, European patent publ. 0484027; Sessler et al, SPIE, 1426: 318-329 (1991); Madison et al, Brain Research, 522: 90-98 (1990); Polo et al, Inorganica Chimica Acta, 192: 1-3 (1992); and Demas et al, J. Macromol. Sci., A25: 1189-1214 (1988).

Suitable acceptor labels for use in the above methods are materials excitable by singlet oxygen to release energy. The acceptor labels can be chemiluminescence reactants capable of reacting with singlet oxygen such that energy is transferred to an acceptor complex in proximity to the chemiluminescence reactant. Exemplary acceptor labels include Europium and Terbium. These acceptor labels are fluorescence dyes that emit fluoresce in a wavelength range from about 200 nm to about 1000 nm.

Suitable supports for use in the above methods include, for example, beads, nitrocellulose membranes, nylon membranes, and derivatized nylon membranes, and also particles, such as agarose, a dextran-based gel, dipsticks, particulates, microspheres, magnetic particles, test tubes, microtiter wells, SEPHADEX™ (Amersham Pharmacia Biotech, Piscataway NJ, and the like. Immobilization can be by absorption or by covalent attachment. Optionally, antibodies can be joined to a linker molecule, such as biotin for attachment to a surface.

Solvents used to extract alpha-synuclein from tissue samples can decrease the sensitivity of the assay (e.g., 5M guanidine, urea/thiourea/CHAPS, urea/thiourea, 1% SDS, 1% SDS/8M, cell lysis buffer). It is recommended that such solvents be removed or diluted such that they account for less than 1% and preferably less than 0.1% of the buffer used for the assay.

Preferred pairs of monoclonal antibodies for use in a bead-based immunoassay are an antibody binding to an epitope within residues 103-108 of alpha-synuclein as the antibody of a donor complex, an antibody specific for pS129 alpha-synuclein as the antibody of a first acceptor complex, and an antibody binding to an epitope within residues 115-122 of alpha-synuclein as the antibody of the second acceptor complex. The antibody of the second acceptor complex detects alpha-synuclein phosphorylated at serine 129. Such antibodies also detect fragments of alpha-synuclein including residues 103-108 and 115-122 or 129 and any additional residues completing the epitope of the pS129-specific antibody.

A preferred implementation of the method has the donor complex comprising, preferably 4B12, linked to a donor bead via streptavidin-biotin and a donor label, the first acceptor complex comprising, preferably 11A5, linked to a bead as a support and Europium as a first acceptor label, and the second acceptor complex comprising, preferably LB509, linked to a bead as a support and Terbium as a second acceptor label. The sample is contacted first by the first and second complexes followed by the addition of the donor complex. The donor label is excited at 680nm which activates the first acceptor label, Europium, to produce the first signal, if pS129 alpha-synuclein is present in the sample, and which also activates the second acceptor label, Terbium, to produce the second signal if total alpha-synuclein is present in the sample. The Terbium signal is preferably detected first by the Resorufine/Amplex Red FP535 filter and the Europium signal is then detected by the Europium 615 filters.

### IV. Applications

### A. Body Fluids

Detection of pS129 h-asyn in patient samples can be used for diagnosing and monitoring diseases characterized by Lewy bodies or other deposits of alpha-synuclein. Synucleinopathic diseases include Parkinson's disease (PD), dementia with Lewy bodies (DLB), the Lewy body variant of Alzheimer's disease (LBVAD), multiple systems atrophy (MSA), neurodegeneration with brain iron accumulation type-1 (NBIA-1), diffuse Lewy body disease (DLBD), and combined PD and Alzheimer's disease (AD). Suitable patient samples include body fluids, such as blood, CSF, ISF, vitreous humour, saliva, urine, and peritoneal fluid. The presence of a synucleinopathic disease is generally associated with significantly altered levels of pS129 alpha-synuclein in the fluid (typically increased) when compared to the mean values in normal individuals, i.e., individuals not suffering from a synucleinopathic disease. A level is significantly altered if it departs by at least one and preferably at least two standard deviations from the mean level in a population of normal individuals. In some methods, a level of total alpha-synuclein or unphosphorylated alpha-synuclein is also determined and optionally a ratio is calculated between the level of phosphorylated alpha-synuclein and total alpha-synuclein or unphosphorylated alpha-synuclein. Such a ratio generally changes in the same direction as pS129 alpha-synuclein (i.e., increased ratio indicates presence or greater severity of disease).

### B. Cell Culture

In vitro monitoring of pS129 alpha-synuclein in conditioned culture medium from a suitable cell culture can be used for analyzing processing, phosphorylation and secretion of pS129 alpha-synuclein and the effect of potential agents on the same. Monitoring phosphorylation of alpha-synuclein provides a means to identify phosphorylases responsible for the same. Agents that inhibit processing and/or secretion of pS129 alpha-synuclein have pharmacological activity potentially useful for prophylaxis of synucleinopathic disease. Typically, inhibitory activity is determined by comparing levels of pS129 alpha-synuclein in medium from a cell treated with a test agent versus a comparable control cell not treated with the agent.

Suitable cells include cells transfected with nucleic acids encoding alpha-synuclein, preferably, human alpha-synuclein and cells naturally expressing alpha-synuclein, also preferably human. The alpha-synuclein in transfected cells can bear a mutation, such as S129A, S129D, A53T and A20P. Cells include PeakS cells, SY5Y cells, human cortical cells, human neuroglioma cell lines, neuroblastoma cell lines, human HeLa cells, primary human endothelial cells (e.g. HUVEC cells), primary human fibroblasts or lymphoblasts, primary human mixed brain cells (including neurons, astrocytes, and neuroglia), Chinese hamster ovary (CHO) cells, and the like. SY5Y cells are neuronal cells that can be induced to differentiate by treatment with retinoic acid/BDNF (brain derived neurotrophic factor). Transfected cells expressing pS129 alpha-synuclein at higher levels than normal human cells are preferred.

Random libraries of peptides or other compounds can also be screened for suitability. Combinatorial libraries can be produced for many types of compounds that can be synthesized in a step-by-step fashion. Such compounds include polypeptides, beta-turn mimetics, polysaccharides, phospholipids, hormones, prostaglandins, steroids, aromatic compounds, heterocyclic compounds, benzodiazepines, oligomeric N-substituted glycines and oligocarbamates. Large combinatorial libraries of the compounds can be constructed by the encoded synthetic libraries (ESL) method described in Affymax, WO 95/12608, Affymax, WO 93/06121, Columbia University, WO 94/08051, Pharmacopeia, WO 95/35503 and Scripps, WO 95/30642. Peptide libraries can also be generated by phage display methods. See, e.g., Devlin, WO 91/18980. The test compounds are typically administered to the culture medium at a concentration in the range from about 1 nM to 1 mM, usually from about 10 µM to 1 mM. Test compounds which are able to inhibit formation, processing or secretion of alpha-synuclein are candidates for further determinations in transgenic animals and eventually human clinical trials.

### C. Transgenic Animals

The antibodies of the invention and assays for detecting them can also be used to monitor pS129 alpha-synuclein production, phosphorylation and processing in transgenic animal models of disease. Transgenic animal models of Lewy body disease are described by Masliah, et al. Science 287:1265-1269 (2000); Masliah et al., PNAS USA 98:12245-12250 (2001). Alpha synuclein can be analyzed either in body fluids as described above for human samples, or in tissue samples taken directly from the animal (see copending 60/423,012, filed Nov. 1, 2002,. Tissue samples can be classified as Lewy body, particulate fraction and soluble fractions. Simple assays can be performed as for cell culture to screen agents for capacity to inhibit formation of pS129 alpha-synuclein. Typically, the inhibitory activity is determined by comparing the level of pS129 alpha- synuclein thereof in a particularly body fluid or fraction from a tissue sample from a transgenic animal treated with the agent in comparison with the level of pS129 alpha-synuclein in the same body fluid or fraction in a control transgenic animal not treated with the agent. Inhibitory activity is shown by decreased levels of pS129 alpha-synuclein thereof in the treated animal relative to the control.

Tissue samples from the brains of human patients can be subject to similar analyses. However, as obtaining samples from the brains of patient is an undesirably invasive procedure, such analyses are usually confined to cadavers.

### V. Kits

The invention further provides kits including pairs of capture and reporter one or more antibodies of the invention. In some kits the capture antibody is preimmobilized to a solid phase, such as a microtiter dish. Optionally, labeling reagents, such as an antiidiotypic antibody are also included in the kits. The labeling may also include a chart or other correspondence regime correlating levels of measured label with levels of antibodies to alpha-synuclein. The term labeling refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits. The kits can be sold, for example, as research or diagnostic reagents.

Although the invention has been described in detail for purposes of clarity of understanding, it will be obvious that certain modifications may be practiced within the scope of the appended claims. Unless otherwise apparent from the context any embodiment, aspect, feature or step can be used in combination with any other. If the content associated with a citation or accession number of the like should change with time, the version existing at the effective filing date of this application is intended, the effective filing date being the actual filing date or earlier filing date of a priority application disclosing the citation or accession number.

### EXAMPLES

### I. Selection of antibody pairs for the total and pS129 human alpha-synuclein specific assays

The signal to background (S/B) ratio for pairs of antibodies towards detection of either total h-asyn or the pS129 h-asyn protein was tested (Table 2; section to the left). Four human specific antibodies tested in the WB experiment were included in the total h-asyn assay development step. The results suggested that the 4B12 antibody worked best when conjugated to biotin and tested against the syn211 and LB509 antibodies coupled to Europium Acceptor beads (bold figures in Table 2). Notably, pairing the same antibodies in the opposite orientation did not give the same robust readout but were 2-5 fold lower in signal-to-background ratio. Furthermore, as epitopes used to raise the 4B12 and syn 204 antibodies have overlapping amino acid residues (especially at position 103 and 108; see Table 1), when these two antibodies were paired, there was no detectable signal.

The three antibodies specific for pS 129 asyn were tested after they were conjugated to the Europium Acceptor-beads and paired with biotinylated 4B12 and syn204 (Table 2; section to the right). This test revealed that the 4B12 - 11A5 antibody combination representing epitopes 103-108 and 124-134 respectively was far superior (more than 6 fold higher signal-to-background ratio) to other pairs and thus was selected for further development.

**Table 1. Antibodies used for establishing duplex assays**

| clone | Company | catalog # | host species | Clonality | epitope | specific detection of | | |
|---|---|---|---|---|---|---|---|---|
| | | | | | | human | rodent | S129 phos |
| asyn211 | Abcam | ab80627 | mouse | monoclonal | 121-125 | yes | no | no |
| LB509 | Covance | SIG-39725 | mouse | monoclonal | 115-122 | yes | no | no |
| asyn204 | Cell Signaling | 2647BF | mouse | monoclonal | 087-111 | yes | No | no |
| 4B12 | Covance | SIG-39730 | mouse | monoclonal | 103-108 | yes | No | no |
| C-20 | Santa Cruz | sc-7011-RL | rabbit | Polyclonal | C-terminus | yes | Yes | no |
| pSyn#64 | WAKO | 014-20281 | mouse | monoclonal | 124-134 | yes | Yes | yes |
| EP1536Y | Abcam | abl73323 | rabbit | monoclonal | | yes | Yes | yes |
| 11A5 | Prothena | N/A | mouse | monoclonal | 124-134 | yes | Yes | yes |
| MJF-R13 | Abcam | abl76839 | rabbit | monoclonal | | yes | Yes | yes |

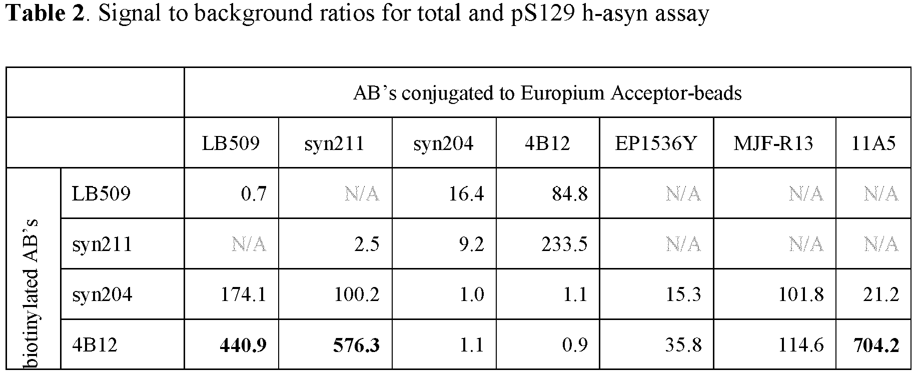

### II. Optimization of the assay condition for uniplex detection of total human alpha-synuclein or pS129 human alpha-synuclein

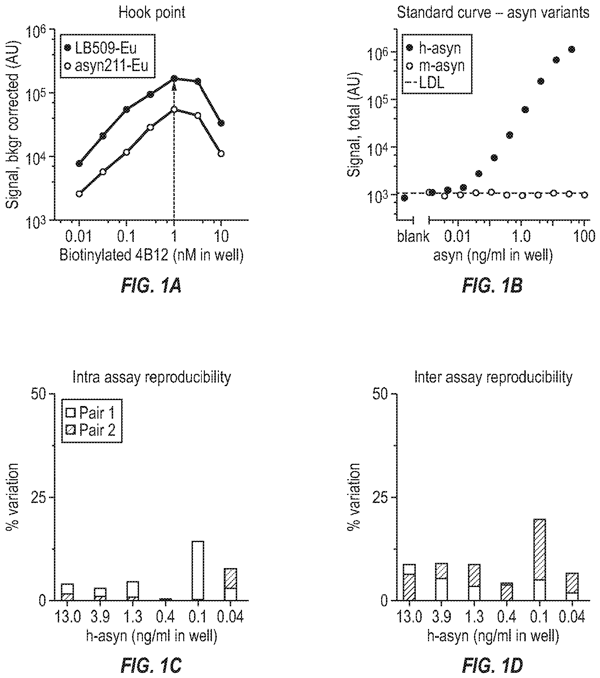

In the case of the total h-asyn assay, the analysis for both the LB509 and syn211 antibodies conjugated to acceptor beads were performed using the biotinylated 4B12 antibody at concentrations between 10pM and 10nM (Fig. 1A). At a concentration of 1.0nM of biotinylated 4B12 both antibody pairs reached their maximum signal. As the signal from biotinylated 4B12 and LB509 coupled Europium Acceptor-bead pair was three times higher at all concentrations tested, this pair was selected as the best condition for detecting total h-asyn.

To evaluate the dose-response characteristics for the total human alpha-synuclein (h-asyn) assay, a standard curve was established using either recombinant GST-tagged h-asyn or recombinant mouse asyn (m-asyn) protein spiked into wild-type rat brain lysate (Fig. 1B). The standard curves for h-asyn displayed a typical sigmoid curve, while the signal for m-asyn did not exceed the lower detection limit (LDL). The linear dynamic range of the h-asyn specific assay lies between 0.1 and 10ng/ml in-well concentration, while the estimated detection limit is about 2.0pg/ml and indicates a 3-times higher sensitivity than what previous asyn assays have reported (Hong et al. Brain 133: 713-726(2010); Emmanouilidou et al. PLoS ONE. (2001); Bidinosti et al. J Biol Chem. 287(40):33691-705(2012)). Intra and inter assay variations were calculated between four individual standard curves using six concentrations on those curves to validate the accuracy and variation between assay runs (Fig. 1C,D) and 22 out of 24 measurements were below 8.7%, with two measurements (0.1ng/ml) at 14.5% and 19.5% (Fig. 1C,D).

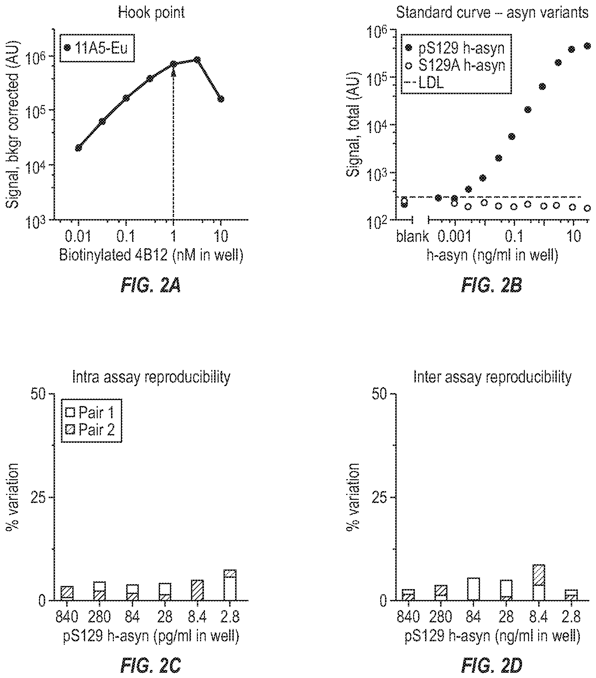

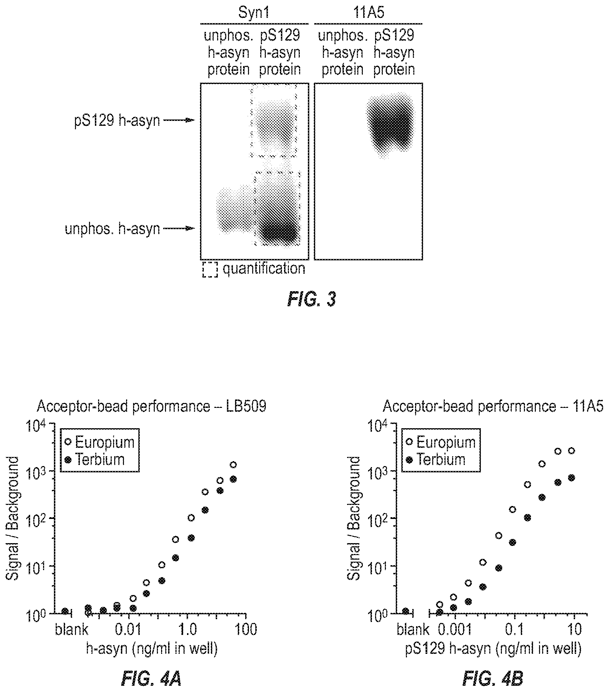

The hook-point analysis for the pS129 h-asyn assay showed that 1.0nM of biotinylated antibody provided the highest signal for the 4B12 - 11A5 antibody pair (Fig. 2A). Dose-response characteristics of the pS129 h-asyn assay was analyzed by establishing standard curves using pS129 h-asyn and S129A h-asyn recombinant protein spiked into asyn knockout mouse brain lysate (Fig. 2B). The standard curve for pS129 h-asyn displayed a typical sigmoidal shape while signal for the S129A h-asyn between 1pg/ml and 100ng/ml concentrations remained below the LDL. To ensure accurate calculations for sensitivity and linear dynamic range of the pS129 h-asyn assay, the amount of phosphorylated h-asyn was measured using isoelectric focusing gels and it was determined 28% of the recombinant protein standard to be phosphorylated (Fig. 3). All values reported for pS129 h-asyn have been adjusted using this factor. The linear dynamic range of the pS129 h-asyn assay for the pS129 h-asyn protein is between 8pg/ml and 3ng/ml in-well concentration with an LDL of 0.3pg/ml, which is about 30-times lower than a previously described pS129 asyn assay (Wang et al. Science Translation Medicine 4:121(2012)). Intra and inter assay variations were again calculated using six different concentrations on four individually run standard curves and was lower than 8.7% for all measurements (Fig. 2C,D).

### III. Feasibility and performance assessment of the duplex assay for quantification of total and pS129 human alpha-synuclein in a single well

The challenge in designing a duplex detection method in an antibody based quantitative assay for a single protein target in two confirmations, as opposed to one where two separate protein targets are measured in the same well, is two-fold: First, a fraction of the target protein is expected to be labeled with two antibodies linked to acceptor beads and a third antibody-biotin complex to anchor the donor bead to the same target. Thus, the assay validation steps have to include assessment of steric hindrance at the binding site of either acceptor-bead coupled antibody by the other. Second, as the two acceptor (reporter) beads rely on the same donor (activator) bead but only one of them is read at a time, the sequence of acquisition of the signal needs to be considered.

In order to design the duplex assay for h-asyn and its post-translationally modified pS129 species, the characteristics of the Terbium Acceptor-beads compared to Europium Acceptor-beads for the LB509 and 11A5 antibodies was evaluated. Signal characteristics of both Acceptor-beads were tested by combining biotinylated 4B12 antibody with LB509 antibody coupled to either Europium or Terbium Acceptor-beads separately and by using purified protein in a dose-response curve. Overall Terbium signal was about 100-fold lower in all measurements including blanks as compared with the Europium signal under identical conditions. As the background signal in this assay was also significantly lower, the S/B ratio of the standard curves obtained using Terbium beads were only marginally affected as compared with the Europium Acceptor-beads (Fig. 4A). The same setup was used for the 4B12 and 11A5 antibody pair (Fig. 4B). In the latter case the difference in S/B between Europium and Terbium Acceptor-beads was more pronounced. Because of this phenomenon and the fact that there will always be a higher quantity of total h-asyn compared to pS129 h-asyn, Terbium Acceptor-beads were chosen coupled to LB509 and conjugated the 11A5 antibody to Europium Acceptor-beads. Thus, reading the Terbium signal first followed by the Europium signal next was more preferable.

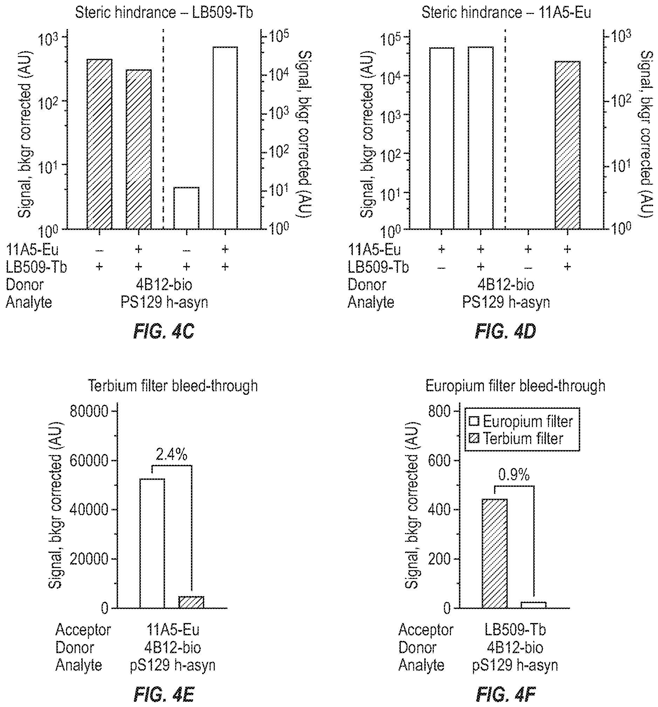

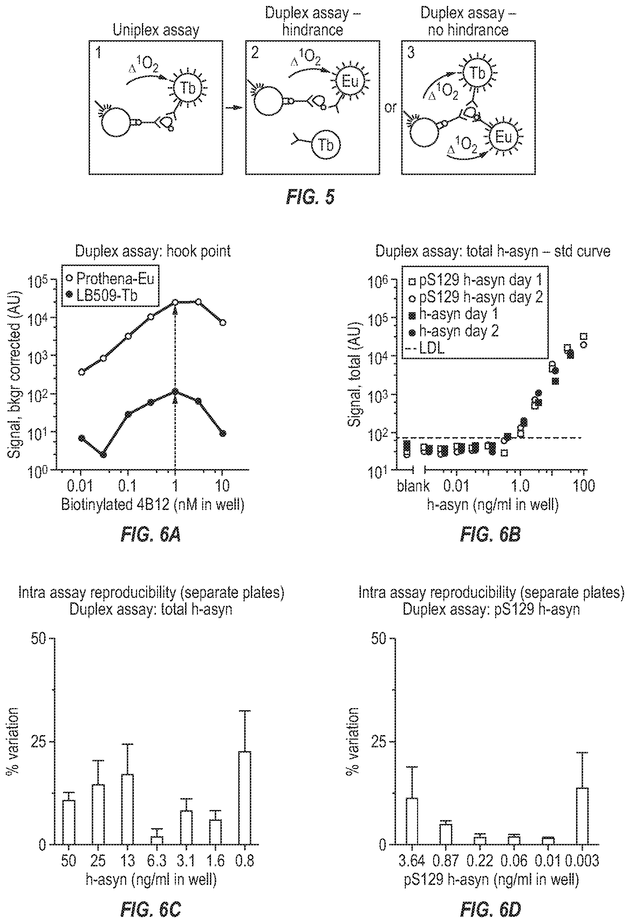

The epitopes of the two Acceptor-bead coupled antibodies in the above assay were very close (see Table 1 for details). Therefore, whether the steric hindrance affected the results by comparing the signal intensity of uniplex and duplex condition for each Acceptor-bead coupled antibody was assessed. When the signal is the same for both conditions, no hindrance occurs when both antibodies are binding to the analyte (Fig. 5). When the signal in the duplex condition is lower than in the uniplex condition, the other Acceptor-bead coupled antibody complex is sterically hindering the binding (Fig. 5). The signal obtained from Terbium Acceptor-bead coupled LB509 (LB509-Tb) [Fig. 4C; where signal detected from the Terbium filter (Resorufine/Amplex Red FP535) for both conditions is displayed on the left, while the signal detected via the Europium filter (Europium 615nm) of the same wells are shown on the right] was not affected due to presence of the 11A5-coupled Europium Acceptor-bead (11A5-Eu). Similarly, the signal obtained from the 11A5-Eu acceptor beads were not influenced by the addition of acceptor beads coupled to the LB509 antibody (Fig. 4D).

Next, to ensure that the signals measured for Terbium and Europium were indeed not influenced by one another, their respective filters for signal bleed-through were tested. Bleed-through can result when Europium and Terbium Acceptor-beads emit signal at the same time but the filter used to separate out the signal of one specific Acceptor-bead also lets light pass from the second Acceptor-bead. Terbium and Europium signals of two different conditions were measured where one condition had no analyte and no second Acceptor-bead (background noise) while in the second condition analyte was added. The signals resulting from those measurements were used to calculate the percent of bleed-through. The bleed-through for the Terbium filter was estimated at 2.4% (Fig. 4E), while the bleed-through of the Europium filter was estimated to be 0.9% (Fig. 4F). All measurements reported for the steric hindrance in this section as well as all other data referring to the duplex assay was corrected for filter bleed-through based on these estimates.

Hook-point was established for the duplex assay where Europium and Terbium signal was measured in the same well. Optimal biotinylated 4B12 concentration was found to be unchanged at 1.0nM (Fig. 6A). To ensure that the Donor-beads added to the wells were sufficient to provide enough oxygen singlets for two Acceptor-beads, a serial dilution of Donor-beads was made ranging from 6µg/ml to 40µg/ml in the well. No change in signal intensity for either Acceptor-bead was seen (data not shown). When comparing the signal intensity of h-asyn and pS129 h-asyn standard curves both measured using the total h-asyn assay (Terbium channel), no difference in signal intensity or curve shape was observed (Fig. 6B). While the LDL of the pS129 h-asyn duplex assay was similar to the LDL obtained for the pS129 h-asyn uniplex assay, a 235-fold increase (from 2pg/ml to 470pg/ml) was observed when comparing the LDL for uniplex and duplex total h-asyn assays.

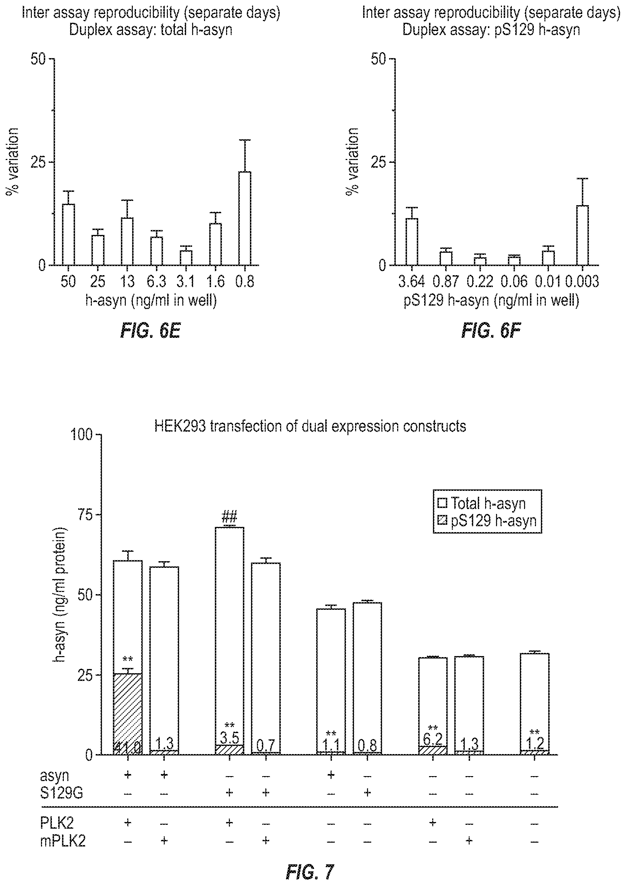

Intra- and inter-assay variations were calculated between six individual standard curves performed on three different days using 6 to 7 concentrations on those curves to validate the accuracy and variation between runs. For the total h-asyn assay, average intra assay variation did not exceed 23% while the average inter assay variation was lower than 22.8% (Fig. 6C,E). Values were notably higher than what was obtained with the uniplex total h-asyn assay. For the pS129 h-asyn assay, average intra-assay variation did not exceed 13.7% while the average inter assay variation was lower than 14.5% (Fig. 6D,F). Z' values were calculated using 12 replicates of naive rodent brain lysate (blank) or lysate spiked with pS129 h-asyn protein. Z' values for the total h-asyn and the pS129 h-asyn assay was 0.81 and 0.84, respectively.

### IV. Quantification of pS129 and total human alpha-synuclein in HEK293 cells co-expressing h-asyn and polo-like kinase 2

To further validate the newly established duplexing assay, HEK293 cells were co-transfected with a single vector construct co-expressing h-asyn and PLK2 under the human CMV and murine CMV promoters, respectively (n=6 per group). PLKs have been shown previously to highly phosphorylate asyn specifically at position 129 (Mbefo et al, J. Biol. Chem. 285:2807-2822 (2010)). As controls, a PLK2 kinase dead-mutant (mPLK2) and non-phosphorylatable S129G h-asyn were used. All proteins were also expressed in combination with YFP or mCherry instead of h-asyn or PLK2, respectively. HEK293 cells produced a substantial amount of endogenous h-asyn as well as pS129 h-asyn (1.2% of total h-asyn), which can be measured using the duplexing assay (Fig. 7). Expression of PLK2 without h-asyn showed an increase in pS129 h-asyn by 4.8 fold due to PLK2 activity compared to single mPLK2 expression but did not phosphorylate h-asyn efficiently/completely. Single h-asyn also resulted in a smaller yet significant increase of pS129 h-asyn compared to expression of S129G h-asyn alone since more phosphorylatable h-asyn is available for endogenous kinases. Expressing S129G h-asyn in combination with PLK2 resulted in an increase in pS129 h-asyn by 5.2-fold due to PLK2 activity on endogenous h-asyn but was considerably smaller (11.7 fold) than in the h-asyn + PLK2 group. When h-asyn is co-expressed with PLK2, pS129 h-asyn levels increased as expected when compared to h-asyn expressed together with mPLK2 (32.4 fold). No difference in total h-asyn levels was observed.

## Claims

1. An in vitro method of simultaneously detecting total alpha-synuclein and S129 phosphorylated (pS129) alpha-synuclein, comprising:
a) contacting a sample with:
(i) a donor complex comprising an antibody that specifically binds to alpha-synuclein linked to a support and a donor label, which is light excitable to activate oxygen to produce singlet oxygen;
(ii) a first acceptor complex for detecting pS129 alpha-synuclein comprising an antibody that binds to pS129 alpha-synuclein, wherein the antibody has an association constant at least five times higher for pS129 alpha-synuclein than for unphosphorylated alpha-synuclein, linked to a support and a first acceptor label; wherein if pS129 alpha-synuclein is present in the sample, the antibodies of the donor complex and first acceptor complex bind to the pS129 alpha-synuclein forming a first sandwich in which the donor label is light excitable to produce singlet oxygen to activate the first acceptor label to produce a first signal;
(iii) a second acceptor complex for detecting total alpha-synuclein comprising an antibody that binds to alpha-synuclein regardless of S129 phosphorylation, the antibody being linked to a support and a second acceptor label; wherein if total alpha-synuclein is present in the sample, the antibodies of the donor complex and second acceptor complex bind to total alpha-synuclein forming a second sandwich in which the donor label is light excitable to produce singlet oxygen to activate the second acceptor label to produce a second signal;
(b) exciting the donor label with a light to activate oxygen to produce singlet oxygen, thereby producing the first signal if pS129 alpha-synuclein is present and the second signal if total alpha-synuclein is present; and
(c) detecting the first and second signals, if any, to indicate presence of pS129 alpha-synuclein and total alpha synuclein, respectively.

2. The method of claim 1, wherein the second signal is detected before the first signal.

3. The method of claim 1 or 2, wherein the contacting of the sample with the donor complex comprises contacting the sample with the support linked to the donor label of the donor complex, wherein the support is further linked to streptavidin and the antibody of the donor complex, wherein the antibody is linked to biotin, wherein the streptavidin binds to the biotin forming the donor complex in the sample to thereby contact the sample.

4. The method of any preceding claim, wherein the sample is contacted with the first and second acceptor complexes and the antibody of the donor complex linked to biotin, incubating the sample, and thereafter contacting the sample with the support further linked to streptavidin, wherein the streptavidin binds to the biotin forming the donor complex in the sample to thereby contact the sample.

5. The method of any preceding claim, performed qualitatively; or performed quantitatively in which the first signal indicates an amount of pS129 alpha-synuclein and the second signal indicates an amount of total alpha-synuclein.

6. The method of any preceding claim, wherein the donor complex, first acceptor complex and second acceptor complex are contacted with the sample simultaneously; or wherein the first and second acceptor complexes are contacted with the sample before the donor complex.

7. The method of any preceding claim, wherein the antibody of the first acceptor complex specifically binds to an epitope of phosphorylated synuclein within residues 123-134, optionally wherein the antibody of the first acceptor complex is 11A5 (ATCC Accession No. PTA-8222).

8. The method of any preceding claim, wherein:
the antibody of the donor complex specifically binds to an epitope within residues 103-108 of alpha-synuclein, optionally wherein the antibody of the donor complex is 4B12; or
wherein the antibody of the donor complex specifically binds to an epitope within residues 40-55 of alpha-synuclein, optionally wherein the antibody of the donor complex is 23E8 (ATCC Accession No. PTA-122711); or
wherein the antibody of the donor complex specifically binds to an epitope within residues 91-97 of alpha-synuclein, optionally wherein the antibody is 1H7 (ATCC Accession No. PTA-8220); or
wherein the antibody of the donor complex specifically binds to an epitope within residues 109-120 of alpha-synuclein, optionally wherein the antibody is 5C12 (ATCC Accession No. PTA-9197); or
wherein the antibody of the donor complex specifically binds to an epitope within residues 115-122 of alpha-synuclein, optionally wherein the antibody is LB509; or
wherein the antibody of the donor complex is 4B12, LB509, 1H7 (ATCC Accession No. PTA-8220), 5C12 (ATCC Accession No. PTA-9197) or 23E8 (ATCC Accession No. PTA-122711).

9. The method of any preceding claim, wherein:
the antibody of the second acceptor complex specifically binds to an epitope within residues 115-122 of alpha-synuclein, optionally wherein the antibody of the second acceptor complex is LB509; or
wherein the antibody of the second acceptor complex specifically binds to an epitope within residues 91-97 of alpha-synuclein, optionally wherein the antibody of the second acceptor complex is 1H7 (ATCC Accession No. PTA-8220); or
wherein the antibody of the second acceptor complex specifically binds to an epitope within residues 109-122 of alpha-synuclein, optionally wherein the antibody of the second acceptor complex is 5C12 (ATCC Accession No. PTA-9197); or
wherein the antibody of the second acceptor complex specifically binds to an epitope within residues 40-55 of alpha-synuclein, optionally wherein the antibody is 23E8 (ATCC Accession No. PTA-122711); or
wherein the antibody of the second acceptor complex is 4B12, LB509, 1H7 (ATCC Accession No. PTA-8220), 5C12 (ATCC Accession No. PTA-9197) or 23E8 (ATCC Accession No. PTA-122711).

10. The method of claim 9, wherein the antibody of the donor complex is 4B12 and the antibody of the second acceptor complex is LB509, 1H7 (ATCC Accession No. PTA-8220), 5C12 (ATCC Accession No. PTA-9197), or 23E8 (ATCC Accession No. PTA-122711); or
wherein the antibody of the donor complex is 23E8 (ATCC Accession No. PTA-122711) and the antibody of the second acceptor complex is LB509, 1H7 (ATCC Accession No. PTA-8220), 5C12 (ATCC Accession No. PTA-9197), or 4B12; or
wherein the antibody of the donor complex is 5C12 (ATCC Accession No. PTA-9197) and the antibody of the second acceptor complex is 23E8 (ATCC Accession No. PTA-122711) or 1H7 (ATCC Accession No. PTA-8220).

11. The method of any preceding claim, wherein the antibody of the donor complex is 4B12, the antibody of the first acceptor complex is 11A5 (ATCC Accession No. PTA-8222), and the antibody of the second acceptor complex is LB509.

12. The method of any preceding claim, wherein the supports of the donor complex, the first acceptor complex, and the second acceptor complex are beads.

13. The method of any preceding claim, wherein the first acceptor label comprises Europium, Terbium, Samarium, or Gadolinium; and/or wherein the second acceptor label comprises Europium, Terbium, Samarium, or Gadolinium;
optionally wherein the first acceptor label comprises Europium and the second acceptor label comprises Terbium.

14. The method of any preceding claim, wherein
(i) the sample is a tissue from a human; or
(ii) the sample is from a transgenic mouse with a transgene expressing human alpha-synuclein.

15. The method of any preceding claim, wherein the sample is a body fluid, optionally cerebrospinal fluid (CSF) of a human.

16. The method of any preceding claim, wherein the sample is a brain, skin, or other organ homogenate of a human, a wild type or transgenic animal; or
wherein the sample is a medium used to culture cells, optionally wherein the cells express recombinant human alpha-synuclein.

## Patentansprüche

1. In-vitro-Verfahren zum gleichzeitigen Nachweis von Gesamt-Alpha-Synuclein und S129-phosphoryliertem (pS129) Alpha-Synuclein, umfassend:
a) Inkontaktbringen einer Probe mit:
(i) einem Donorkomplex, umfassend einen Antikörper, der spezifisch an Alpha-Synuclein bindet, das an einen Träger gebunden ist, und eine Donormarkierung, die lichtanregbar ist, um Sauerstoff zu aktivieren, um Singulettsauerstoff zu erzeugen;
(ii) einem ersten Akzeptorkomplex zum Nachweis von pS129-Alpha-Synuclein, umfassend einen Antikörper, der an pS129-Alpha-Synuclein bindet, wobei der Antikörper eine mindestens fünfmal höhere Assoziationskonstante für pS129-Alpha-Synuclein aufweist als für nichtphosphoryliertes Alpha-Synuclein, gebunden an einen Träger und eine erste Akzeptormarkierung; wobei, wenn pS129-Alpha-Synuclein in der Probe vorhanden ist, die Antikörper des Donorkomplexes und des ersten Akzeptorkomplexes an das pS129-Alpha-Synuclein binden und ein erstes Sandwich bilden, in dem die Donormarkierung lichtanregbar ist, um Singulettsauerstoff zu erzeugen, um die erste Akzeptormarkierung zu aktivieren, um ein erstes Signal zu erzeugen;
(iii) einem zweiten Akzeptorkomplex zum Nachweis des Gesamt-Alpha-Synucleins, umfassend einen Antikörper, der unabhängig von der S129-Phosphorylierung an Alpha-Synuclein bindet, wobei der Antikörper an einen Träger und eine zweite Akzeptormarkierung gebunden ist; wobei, wenn Gesamt-Alpha-Synuclein in der Probe vorhanden ist, die Antikörper des Donorkomplexes und des zweiten Akzeptorkomplexes an Gesamt-Alpha-Synuclein binden und ein zweites Sandwich bilden, in dem die Donormarkierung lichtanregbar ist, um Singulettsauerstoff zu erzeugen, um die zweite Akzeptormarkierung zu aktivieren, um ein zweites Signal zu erzeugen;
(b) Anregen der Donormarkierung mit einem Licht zum Aktivieren von Sauerstoff zum Erzeugen von Singulettsauerstoff, wodurch das erste Signal erzeugt wird, wenn pS129-Alpha-Synuclein vorhanden ist, und das zweite Signal, wenn Gesamt-Alpha-Synuclein vorhanden ist; und
(c) Erfassen des ersten und zweiten Signals, falls vorhanden, um das Vorhandensein von pS129-Alpha-Synuclein bzw. Gesamt-Alpha-Synuclein anzuzeigen.

2. Verfahren nach Anspruch 1, wobei das zweite Signal vor dem ersten Signal erfasst wird.

3. Verfahren nach Anspruch 1 oder 2, wobei das Inkontaktbringen der Probe mit dem Donorkomplex das Inkontaktbringen der Probe mit dem Träger umfasst, der mit der Donormarkierung des Donorkomplexes verbunden ist, wobei der Träger ferner mit Streptavidin und dem Antikörper des Donorkomplexes verbunden ist und wobei der Antikörper an Biotin gebunden ist, wobei das Streptavidin an das Biotin bindet, das den Donorkomplex in der Probe bildet, um dadurch die Probe zu kontaktieren.

4. Verfahren nach einem der vorhergehenden Ansprüche, wobei die Probe mit dem ersten und zweiten Akzeptorkomplex und dem Antikörper des an Biotin gebundenen Donorkomplexes in Kontakt gebracht wird, die Probe inkubiert wird und danach die Probe mit dem ferner mit Streptavidin verbundenen Träger in Kontakt gebracht wird, wobei das Streptavidin an das Biotin bindet, das den Donorkomplex in der Probe bildet, um dadurch die Probe zu kontaktieren.

5. Verfahren nach einem der vorhergehenden Ansprüche, qualitativ durchgeführt; oder
quantitativ durchgeführt, wobei das erste Signal eine Menge an pS129-Alpha-Synuclein und das zweite Signal eine Menge an Gesamt-Alpha-Synuclein anzeigt.

6. Verfahren nach einem der vorhergehenden Ansprüche, wobei der Donorkomplex, der erste Akzeptorkomplex und der zweite Akzeptorkomplex gleichzeitig mit der Probe in Kontakt gebracht werden; oder
wobei der erste und der zweite Akzeptorkomplex mit der Probe vor dem Donorkomplex in Kontakt gebracht werden.

7. Verfahren nach einem der vorhergehenden Ansprüche, wobei der Antikörper des ersten Akzeptorkomplexes spezifisch an ein Epitop von phosphoryliertem Synuclein innerhalb der Reste 123-134 bindet, wobei der Antikörper des ersten Akzeptorkomplexes optional 11A5 (ATCC-Zugangsnummer PTA-8222) ist.

8. Verfahren nach einem der vorhergehenden Ansprüche, wobei:
der Antikörper des Donorkomplexes spezifisch an ein Epitop innerhalb der Reste 103-108 von Alpha-Synuclein bindet, wobei der Antikörper des Donorkomplexes optional 4B12 ist; oder
wobei der Antikörper des Donorkomplexes spezifisch an ein Epitop innerhalb der Reste 40-55 von Alpha-Synuclein bindet, wobei der Antikörper des Donorkomplexes optional 23E8 (ATCC-Zugangsnummer PTA-122711) ist; oder
wobei der Antikörper des Donorkomplexes spezifisch an ein Epitop innerhalb der Reste 91-97 von Alpha-Synuclein bindet, wobei der Antikörper optional 1H7 (ATCC-Zugangsnummer PTA-8220) ist; oder
wobei der Antikörper des Donorkomplexes spezifisch an ein Epitop innerhalb der Reste 109-120 von Alpha-Synuclein bindet, wobei der Antikörper optional 5C12 (ATCC-Zugangsnummer PTA-9197) ist; oder
wobei der Antikörper des Donorkomplexes spezifisch an ein Epitop innerhalb der Reste 115-122 von Alpha-Synuclein bindet, wobei der Antikörper optional LB509 ist; oder
wobei der Antikörper des Donorkomplexes 4B12, LB509, 1H7 (ATCC-Zugangsnummer PTA-8220), 5C12 (ATCC-Zugangsnummer PTA-9197) oder 23E8 (ATCC-Zugangsnummer PTA-122711) ist.

9. Verfahren nach einem der vorhergehenden Ansprüche, wobei:
der Antikörper des zweiten Akzeptorkomplexes spezifisch an ein Epitop innerhalb der Reste 115-122 von Alpha-Synuclein bindet, wobei der Antikörper des zweiten Akzeptorkomplexes optional LB509 ist; oder
wobei der Antikörper des zweiten Akzeptorkomplexes spezifisch an ein Epitop innerhalb der Reste 91-97 von Alpha-Synuclein bindet, wobei der Antikörper des zweiten Akzeptorkomplexes optional 1H7 (ATCC-Zugangsnummer PTA-8220) ist; oder
wobei der Antikörper des zweiten Akzeptorkomplexes spezifisch an ein Epitop innerhalb der Reste 109-122 von Alpha-Synuclein bindet, wobei der Antikörper des zweiten Akzeptorkomplexes optional 5C12 (ATCC-Zugangsnummer PTA-9197) ist; oder
wobei der Antikörper des zweiten Akzeptorkomplexes spezifisch an ein Epitop innerhalb der Reste 40-55 von Alpha-Synuclein bindet, wobei der Antikörper optional 23E8 (ATCC-Zugangsnummer PTA-122711) ist; oder
wobei der Antikörper des zweiten Akzeptorkomplexes 4B12, LB509, 1H7 (ATCC-Zugangsnummer PTA-8220), 5C12 (ATCC-Zugangsnummer PTA-9197) oder 23E8 (ATCC-Zugangsnummer PTA-122711) ist.

10. Verfahren nach Anspruch 9, wobei der Antikörper des Donorkomplexes 4B12 und der Antikörper des zweiten Akzeptorkomplexes LB509, 1H7 (ATCC-Zugangsnummer PTA-8220), 5C12 (ATCC-Zugangsnummer PTA-9197) oder 23E8 (ATCC-Zugangsnummer PTA-122711) ist; oder
wobei der Antikörper des Donorkomplexes 23E8 (ATCC-Zugangsnummer PTA-122711) und der Antikörper des zweiten Akzeptorkomplexes LB509, 1H7 (ATCC-Zugangsnummer PTA-8220), 5C12 (ATCC-Zugangsnummer PTA-9197) oder 4B12 ist; oder
wobei der Antikörper des Donorkomplexes 5C12 (ATCC-Zugangsnummer PTA-9197) ist und der Antikörper des zweiten Akzeptorkomplexes 23E8 (ATCC-Zugangsnummer PTA-122711) oder 1H7 (ATCC-Zugangsnummer PTA-8220) ist.

11. Verfahren nach einem der vorhergehenden Ansprüche, wobei der Antikörper des Donorkomplexes 4B12 ist, der Antikörper des ersten Akzeptorkomplexes 11A5 (ATCC-Zugangsnummer PTA-8222) ist und der Antikörper des zweiten Akzeptorkomplexes LB509 ist.

12. Verfahren nach einem der vorhergehenden Ansprüche, wobei die Träger des Donorkomplexes, des ersten Akzeptorkomplexes und des zweiten Akzeptorkomplexes Kügelchen sind.

13. Verfahren nach einem der vorhergehenden Ansprüche, wobei die erste Akzeptormarkierung Europium, Terbium, Samarium oder Gadolinium umfasst; und/oder wobei die zweite Akzeptormarkierung Europium, Terbium, Samarium oder Gadolinium umfasst;
wobei die erste Akzeptormarkierung optional Europium umfasst und die zweite Akzeptormarkierung Terbium umfasst.

14. Verfahren nach einem der vorhergehenden Ansprüche, wobei
(i) die Probe ein Gewebe eines Menschen ist; oder
(ii) die Probe von einer transgenen Maus stammt, wobei ein Transgen menschliches Alpha-Synuclein exprimiert.

15. Verfahren nach einem der vorhergehenden Ansprüche, wobei die Probe eine Körperflüssigkeit, gegebenenfalls Cerebrospinalflüssigkeit (CSF) eines Menschen ist.

16. Verfahren nach einem der vorhergehenden Ansprüche, wobei die Probe ein Gehirn-, Haut- oder ein anderes Organhomogenisat eines Menschen, eines Wildtyp- oder eines transgenen Tieres ist; oder
wobei die Probe ein Medium ist, das zum Kultivieren von Zellen verwendet wird, wobei die Zellen optional rekombinantes menschliches Alpha-Synuclein exprimieren.

## Revendications

1. Procédé in vitro de détection simultanée de l'alpha-synucléine totale et de l'alpha-synucléine phosphorylée par S129 (pS129), comprenant :
a) la mise en contact d'un échantillon avec :
(i) un complexe donneur comprenant un anticorps qui se lie spécifiquement à l'alpha-synucléine reliée à un support et à un marqueur donneur, qui est excitable à la lumière pour activer de l'oxygène afin de produire de l'oxygène singulet ;
(ii) un premier complexe accepteur destiné à détecter l'alpha-synucléine pS129 comprenant un anticorps qui se lie à l'alpha-synucléine pS129, l'anticorps ayant une constante d'association au moins cinq fois plus élevée pour l'alpha-synucléine pS129 que pour l'alpha-synucléine non phosphorylée, reliée à un support et à un premier marqueur accepteur ; si l'alpha-synucléine pS129 est présente dans l'échantillon, les anticorps du complexe donneur et du premier complexe accepteur se liant à l'alpha-synucléine pS129 formant un premier intercalage dans lequel le marqueur donneur est excitable à la lumière pour produire de l'oxygène singulet afin d'activer le premier marqueur accepteur de manière à produire un premier signal ;
(iii) un second complexe accepteur destiné à détecter l'alpha-synucléine totale comprenant un anticorps qui se lie à l'alpha-synucléine indépendamment de la phosphorylation par S129, l'anticorps étant relié à un support et à un second marqueur accepteur ; si l'alpha-synucléine totale est présente dans l'échantillon, les anticorps du complexe donneur et du second complexe accepteur se liant à l'alpha-synucléine totale formant un second intercalage dans lequel le marqueur donneur est excitable à la lumière pour produire de l'oxygène singulet afin d'activer le second marqueur accepteur de manière à produire un second signal ;
(b) l'excitation du marqueur donneur avec une lumière pour activer de l'oxygène afin de produire de l'oxygène singulet, produisant ainsi le premier signal si l'alpha-synucléine pS129 est présente et le second signal si l'alpha-synucléine totale est présente ; et
(c) la détection des premier et second signaux, le cas échéant, pour indiquer la présence de l'alpha-synucléine pS129 et de l'alpha-synucléine totale, respectivement.

2. Procédé selon la revendication 1, le second signal étant détecté avant le premier signal.

3. Procédé selon la revendication 1 ou 2, la mise en contact de l'échantillon avec le complexe donneur comprenant la mise en contact de l'échantillon avec le support relié au marqueur donneur du complexe donneur, le support étant en outre relié à la streptavidine et à l'anticorps du complexe donneur, l'anticorps étant relié à la biotine, la streptavidine se liant à la biotine formant le complexe donneur dans l'échantillon pour ainsi entrer en contact avec l'échantillon.

4. Procédé selon l'une quelconque des revendications précédentes, l'échantillon étant mis en contact avec les premier et second complexes accepteurs et l'anticorps du complexe donneur relié à la biotine, incubant l'échantillon, puis mettant en contact l'échantillon avec le support en outre relié à la streptavidine, la streptavidine se liant à la biotine formant le complexe donneur dans l'échantillon pour ainsi entrer en contact avec l'échantillon.

5. Procédé selon l'une quelconque des revendications précédentes, exécuté qualitativement ; ou
exécuté quantitativement dans lequel le premier signal indique une quantité d'alpha-synucléine pS129 et le second signal indique une quantité d'alpha-synucléine totale.

6. Procédé selon l'une quelconque des revendications précédentes, le complexe donneur, le premier complexe accepteur et le second complexe accepteur étant mis en contact avec l'échantillon simultanément ; ou
les premier et second complexes accepteurs étant mis en contact avec l'échantillon avant le complexe donneur.

7. Procédé selon l'une quelconque des revendications précédentes, l'anticorps du premier complexe accepteur se liant spécifiquement à un épitope de synucléine phosphorylée à l'intérieur des résidus 123 à 134, éventuellement l'anticorps du premier complexe accepteur étant 11A5 (n° d'accès ATCC PTA-8222).

8. Procédé selon l'une quelconque des revendications précédentes :
l'anticorps du complexe donneur se liant spécifiquement à un épitope à l'intérieur des résidus 103 à 108 de l'alpha-synucléine, éventuellement l'anticorps du complexe donneur étant 4B12 ; ou
l'anticorps du complexe donneur se liant spécifiquement à un épitope à l'intérieur des résidus 40 à 55 de l'alpha-synucléine, éventuellement l'anticorps du complexe donneur étant 23E8 (n° d'accès ATCC PTA-122711) ; ou
l'anticorps du complexe donneur se liant spécifiquement à un épitope à l'intérieur des résidus 91 à 97 de l'alpha-synucléine, éventuellement l'anticorps étant 1H7 (n° d'accès ATCC PTA-8220) ; ou
l'anticorps du complexe donneur se liant spécifiquement à un épitope à l'intérieur des résidus 109 à 120 de l'alpha-synucléine, éventuellement l'anticorps étant 5C12 (n° d'accès ATCC PTA-9197) ; ou
l'anticorps du complexe donneur se liant spécifiquement à un épitope à l'intérieur des résidus 115 à 122 de l'alpha-synucléine, éventuellement l'anticorps étant LB509 ; ou
l'anticorps du complexe donneur étant 4B12, LB509, 1H7 (n° d'accès ATCC PTA-8220), 5C12 (n° d'accès ATCC PTA-9197) ou 23E8 (n° d'accès ATCC PTA-122711).

9. Procédé selon l'une quelconque des revendications précédentes :
l'anticorps du second complexe accepteur se liant spécifiquement à un épitope à l'intérieur des résidus 115 à 122 de l'alpha-synucléine, éventuellement l'anticorps du second complexe accepteur étant LB509 ; ou
l'anticorps du second complexe accepteur se liant spécifiquement à un épitope à l'intérieur des résidus 91 à 97 de l'alpha-synucléine, éventuellement l'anticorps du second complexe accepteur étant 1H7 (n° d'accès ATCC PTA-8220) ; ou
l'anticorps du second complexe accepteur se liant spécifiquement à un épitope à l'intérieur des résidus 109 à 122 de l'alpha-synucléine, éventuellement l'anticorps du second complexe accepteur étant 5C12 (n° d'accès ATCC PTA-9197) ; ou
l'anticorps du second complexe accepteur se liant spécifiquement à un épitope à l'intérieur des résidus 40 à 55 de l'alpha-synucléine, éventuellement l'anticorps étant 23E8 (n° d'accès ATCC PTA-122711) ; ou
l'anticorps du second complexe accepteur étant 4B12, LB509, 1H7 (n° d'accès ATCC PTA-8220), 5C12 (n° d'accès ATCC PTA-9197) ou 23E8 (n° d'accès ATCC PTA-122711).

10. Procédé selon la revendication 9, l'anticorps du complexe donneur étant 4B12 et l'anticorps du second complexe accepteur étant LB509, 1H7 (n° d'accès ATCC PTA-8220), 5C12 (n° d'accès ATCC PTA-9197) ou 23E8 (n° d'accès ATCC PTA-122711) ; ou
l'anticorps du complexe donneur étant 23E8 (n° d'accès ATCC PTA-122711)
et l'anticorps du second complexe accepteur étant LB509, 1H7 (n° d'accès ATCC PTA-8220), 5C12 (n° d'accès ATCC PTA-9197), ou 4B12 ; ou
l'anticorps du complexe donneur étant 5C12 (n° d'accès ATCC PTA-9197)_et l'anticorps du second complexe accepteur étant 23E8 (n° d'accès ATCC PTA-122711)_ou 1H7 (n° d'accès ATCC PTA-8220).

11. Procédé selon l'une quelconque des revendications précédentes, l'anticorps du complexe donneur étant 4B12, l'anticorps du premier complexe accepteur étant 11A5 (n° d'accès ATCC PTA-8222) et l'anticorps du second complexe accepteur étant LB509.

12. Procédé selon l'une quelconque des revendications précédentes, les supports du complexe donneur, du premier complexe accepteur et du second complexe accepteur étant des billes.

13. Procédé selon l'une quelconque des revendications précédentes, le premier marqueur accepteur comprenant de l'europium, du terbium, du samarium ou du gadolinium ; et/ou le second marqueur accepteur comprenant de l'europium, du terbium, du samarium ou du gadolinium ;
éventuellement le premier marqueur accepteur comprenant de l'europium et le second marqueur accepteur comprenant du terbium.

14. Procédé selon l'une quelconque des revendications précédentes,
(i) l'échantillon étant un tissu provenant d'un être humain ; ou
(ii) l'échantillon provenant d'une souris transgénique avec un transgène exprimant l'alpha-synucléine humaine.

15. Procédé selon l'une quelconque des revendications précédentes, l'échantillon étant un fluide corporel, éventuellement du liquide céphalorachidien (LCR) d'un être humain.

16. Procédé selon l'une quelconque des revendications précédentes, l'échantillon étant un homogénat de cerveau, de peau ou d'un autre organe d'un être humain, d'un animal de type sauvage ou transgénique ; ou
l'échantillon étant un milieu utilisé pour cultiver des cellules, éventuellement les cellules exprimant l'alpha-synucléine humaine recombinante.


### SEQUENCE LISTING

<110> PROTHENA BIOSCIENCES LIMITED
   Barbour, Robin
   Kirik, Deniz
   Landeck, Natalie Regina
<120> Assay for Detecting Total and S129
   Phosphorylated Alpha-Synuclein
<130> 057450/497105
<150> 62/359,139
   <151> 07/06/2016
<160> 1
<170> FastSEQ for Windows Version 4.0
<210> 1
   <211> 140
   <212> PRT
   <213> homo sapiens
<400> 1